# EUROPEAN PATENT APPLICATION

(11) **EP 4 257 672 A1**
(43) Date of publication of application: **11.10.2023**
(21) Application number: 21900710.1
(22) Date of filing: 03.12.2021
(51) Int. Cl.: C12N 5/071, C12Q 1/06

(54) **METHOD FOR PRODUCING TWO-DIMENSIONAL SMALL INTESTINAL ORGANOIDS HAVING VILLUS STRUCTURE**

(30) Priority: 03.12.2020 JP 2020201332
(71) Applicant: Keio University, Tokyo, 108-8345 (JP); JSR Corporation, Tokyo 105-8640 (JP)
(72) Inventor: SATO Toshiro, Tokyo 160-8582 (JP); SUGIMOTO Shinya, Tokyo 160-8582 (JP); ARAI Kazuya, Tokyo 105-8640 (JP)
(74) Representative: TBK
(86) International application number: PCT/JP2021/044494
(87) International publication number: WO 2022/118961

(57) **Abstract**

A method for producing a two-dimensional small intestinal organoid having a villus structure, the method including a step 1 of culturing a cell derived from a small intestinal epithelium in an extracellular matrix to obtain a three-dimensional small intestinal organoid, a step 2 of dispersing the three-dimensional small intestinal organoid and monolayer culturing on the extracellular matrix to obtain a two-dimensional small intestinal organoid, and a step 3 of further culturing the two-dimensional small intestinal organoid while letting a culture medium of the two-dimensional small intestinal organoid to flow so that the two-dimensional small intestinal organoid forms a villus structure.

## Description

### [Technical Field]

The present invention relates to a method for producing a two-dimensional small intestinal organoid having a villus structure. In more detail, the present invention relates to a method for producing a two-dimensional small intestinal organoid having a villus structure, a two-dimensional small intestinal organoid having a villus structure, elucidation of the mechanism of small intestinal diseases, a method for screening an absorption controller of a nutrient or a drug in the small intestine, a method for screening a formation controller of the villus structure in the small intestine, a method for screening a regulator of the digestion, absorption, or metabolism of a target substance in the small intestine, a method for screening a substance that regulates the growth of an intestinal bacterium and/or a virus in the small intestine, and a transplant material. Priority is claimed on Japanese Patent Application No. 2020-201332, filed December 3, 2020, the content of which is incorporated herein by reference. In addition, the present application was published in the following paper (Sugimoto S., et al., An organoid-based organ-repurposing approach to treat short bowel syndrome, Nature, 592 (7852), 99-104, 2021.) on February 24, 2021, the content of which is incorporated herein by reference.

### [Background Art]

Methods for producing a three-dimensional organoid using an intestinal tissue such as a normal small intestinal organoid, a gastrointestinal tumor organoid, or a colon organoid are known (for example, refer to Patent Documents 1 and 2). In addition, the inventors have previously developed a technique for producing a two-dimensional organoid by dispersing a three-dimensional organoid derived from a human intestinal epithelium and culturing the organoid in a monolayer (for example, refer to Patent Document 3).

### [Citation List]

### [Patent Documents]

[Patent Document 1] International Publication No. WO 2017/142069
[Patent Document 2] International Publication No. WO 2017/199811
[Patent Document 3] International Publication No. WO 2018/038042

### [Summary of Invention]

### [Technical Problem]

According to the method described in Patent Document 3, a two-dimensional organoid containing a cell that configures a mature intestinal tract can be produced by producing a three-dimensional organoid imitating a mature intestinal tissue from an intestinal tissue and dispersing the three-dimensional organoid to culture the organoid in two dimensions.

The two-dimensional organoid described in Patent Document 3 has a structure in which the cell that configures the mature intestinal tract spreads on a two-dimensional plane and is capable of infecting and growing viruses that infect intestinal tissues such as a norovirus.

However, the two-dimensional organoid described in Patent Document 3 has a flat structure and does not have a villus structure that provides a function such as nutrient absorption, which is a physiological function of the intestinal tract. Therefore, it was difficult to investigate the mechanism relating to the original intestinal lumen structure, such as absorption in the intestinal tract, using the two-dimensional organoid described in Patent Document 3.

Therefore, an object of the present invention is to provide a technique for producing a two-dimensional small intestinal organoid having a villus structure.

### [Solution to Problem]

The present invention includes the following aspects.
[1] A method for producing a two-dimensional small intestinal organoid having a villus structure, the method including a step 1 of culturing a cell derived from a small intestinal epithelium in an extracellular matrix to obtain a three-dimensional small intestinal organoid, a step 2 of dispersing the three-dimensional small intestinal organoid and monolayer culturing on the extracellular matrix to obtain a two-dimensional small intestinal organoid, and a step 3 of further culturing the two-dimensional small intestinal organoid while letting a culture medium of the two-dimensional small intestinal organoid to flow so that the two-dimensional small intestinal organoid forms a villus structure.
[2] A two-dimensional small intestinal organoid having a villus structure.
[3] The two-dimensional small intestinal organoid according to [2], in which the two-dimensional small intestinal organoid is produced from a cell derived from a patient with a small intestinal dysfunction or a small intestinal disease.
[4] The two-dimensional small intestinal organoid according to [2], in which the two-dimensional small intestinal organoid is produced from a cell derived from a normal small intestine.
[5] A method for screening an absorption controller of a nutrient or a drug in a small intestine, the method including a step of measuring an absorption amount of a nutrient or a drug by a two-dimensional small intestinal organoid having a villus structure in a presence of a test substance, in which a case where the absorption amount is significantly changed compared with that in an absence of the test substance indicates that the test substance is an absorption controller of a nutrient or a drug in a small intestine.
[6] A method for screening a formation controller of a villus structure in a small intestine, the method including a step of producing a two-dimensional small intestinal organoid having a villus structure in a presence of a test substance and a step of evaluating a morphology of the villus structure of the produced two-dimensional small intestinal organoid, in which a case where the morphology is significantly changed compared with that in an absence of the test substance indicates that the test substance is a formation controller of a villus structure in a small intestine.
[7] A method for screening a regulator of digestion, absorption, or metabolism of a target substance in a small intestine, the method including a step of producing a two-dimensional small intestinal organoid having a villus structure in a presence of a test substance and a step of measuring digestion, absorption, or metabolism of a target substance in the produced two-dimensional small intestinal organoid to obtain a measurement value, in which a case where the measurement value is significantly changed compared with that in an absence of the test substance indicates that the test substance is a regulator of digestion, absorption, or metabolism of the target substance in a small intestine.
[8] A method for screening a substance that regulates growth of an intestinal bacterium and/or a virus in a small intestine, the method including a step of measuring growth of an intestinal bacterium and/or a virus in a two-dimensional small intestinal organoid having a villus structure in a presence of a test substance, in which a case where the growth of the intestinal bacterium and/or virus is significantly changed compared with that in an absence of the test substance indicates that the test substance is a substance that regulates growth of an intestinal bacterium and/or a virus in a small intestine.
[9] A transplant material containing a small intestinal organoid, the transplant material is for being transplanted into a large intestine of a living body to cause an intestinal fluid to flow so that a tissue having a small intestinal function is formed in the large intestine.

### [Advantageous Effects of Invention]

According to the present invention, it is possible to provide a technique for producing a two-dimensional small intestinal organoid having a villus structure and an organoid for a small intestinal disease treatment.

### [Brief Description of Drawings]

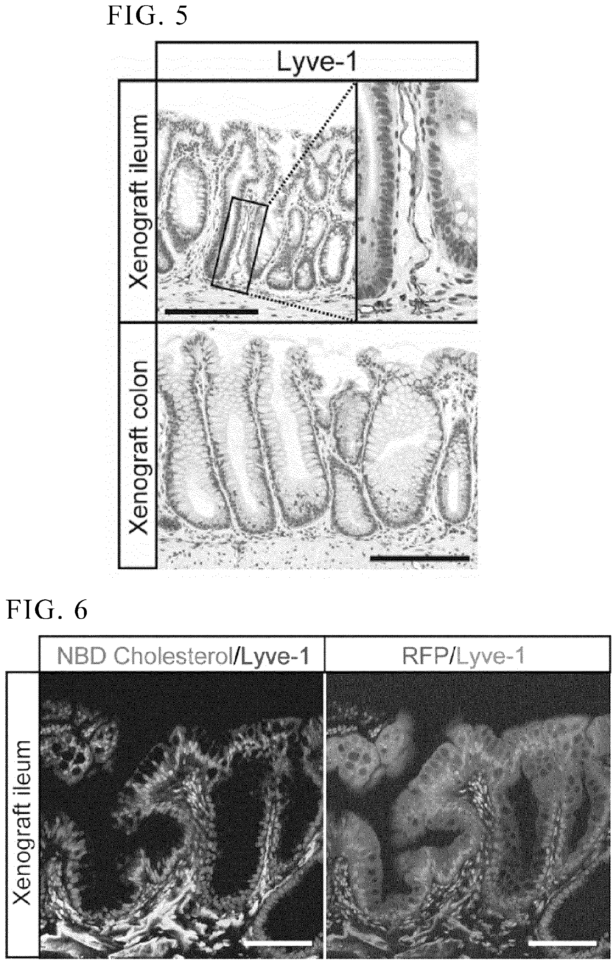

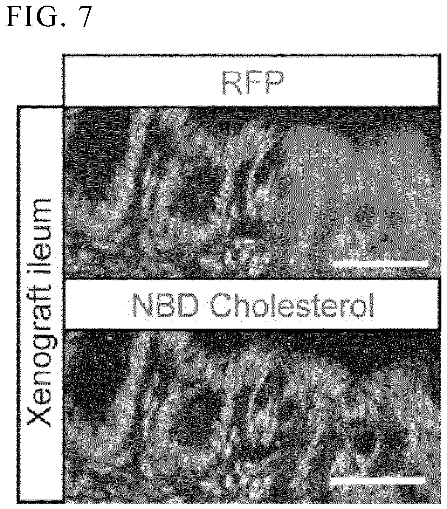

FIG. 1 is a schematic view showing a procedure for transplanting human ileal organoids or human colon organoids into the large intestines of immunodeficient mice (NOG mice) in Experimental Example 1.
FIG. 2 is a micrograph showing the result of hematoxylin and eosin staining of a tissue section of a human ileal organoid graft in Experimental Example 1.
FIG. 3 is a transmission electron micrograph of a tissue section of a human ileal organoid graft captured in Experimental Example 1.
FIG. 4 is photographs showing the results of detecting the expression of human cytokeratin and sucrase-isomaltase in human ileal organoid grafts and human colon organoid grafts in Experimental Example 2.
FIG. 5 is photographs showing the results of detecting the expression of a lymphatic vessel endothelial hyaluronan receptor 1 (Lyve-1) in a human ileal organoid graft and a human colon organoid graft in Experimental Example 3.
The left part of FIG. 6 is a photograph showing the result of detecting the presence of NBD-cholesterol and Lyve-1 in a human ileal organoid graft in Experimental Example 3. The right part of FIG. 6 is a photograph showing the result of detecting the presence of RFP and Lyve-1, which shows the presence of a human ileal organoid graft, in the same visual field as in the left part of FIG. 6.
The upper part of FIG. 7 is a photograph showing the result of detecting RFP, which shows the presence of a human ileal organoid graft, in the boundary between a recipient's large intestine and a human ileal organoid graft in Experimental Example 3. The lower part of FIG. 7 is a photograph showing the result of detecting NBD-cholesterol in the same visual field as in the upper part of FIG. 7.

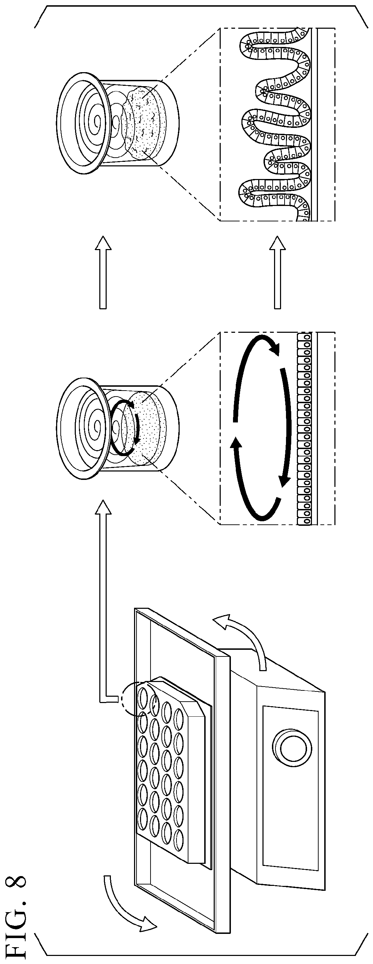

FIG. 8 is a schematic view showing an outline of an experiment in Experimental Example 4.

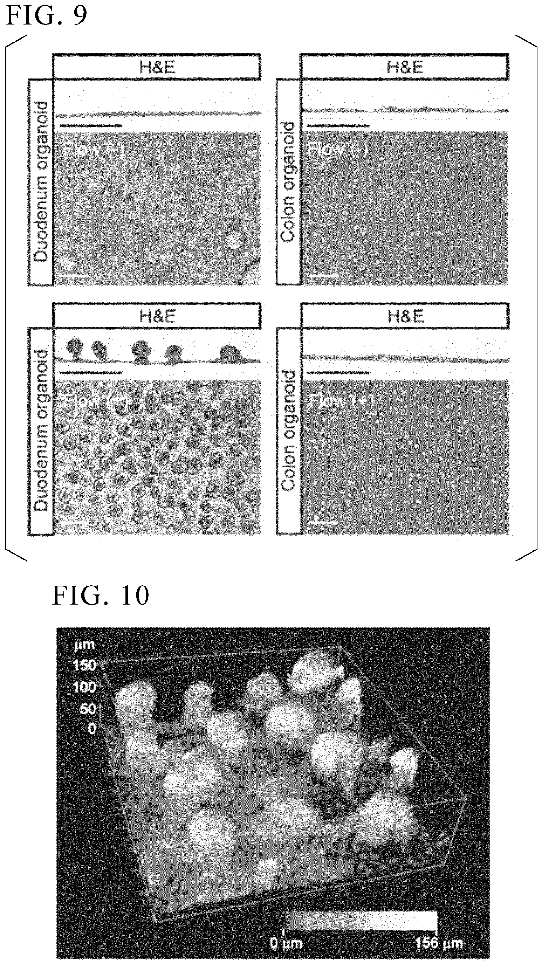

FIG. 9 is micrographs showing the results of culturing a two-dimensionally cultured human duodenal organoid and a two-dimensionally cultured human colon organoid in the presence and absence of the flow of a culture medium in Experimental Example 4.
FIG. 10 is a 3D image showing the result of culturing the two-dimensionally cultured duodenal organoid for 4 days in the presence of the flow of the culture medium, then, fixing the organoid, staining nuclei with Hoechst33342, capturing the nuclei with a two-photon microscope, and performing a coloring process based on the height in Experimental Example 4.

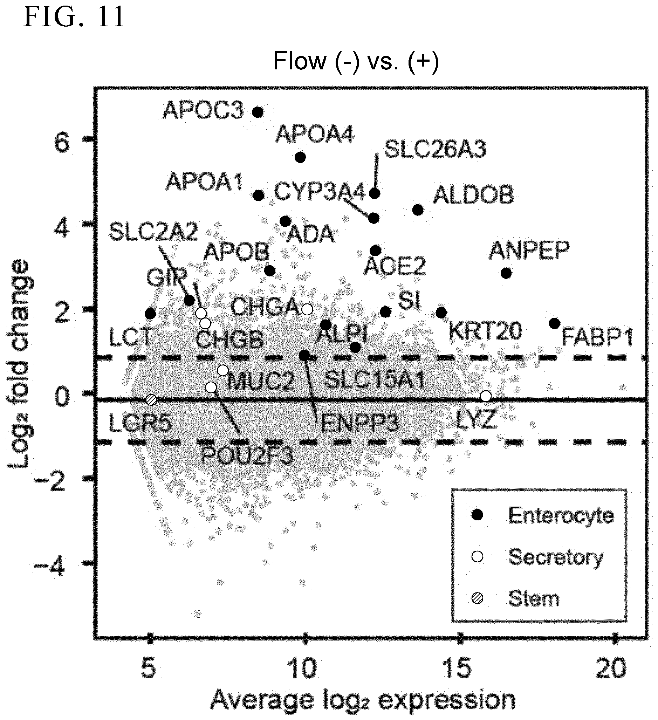

FIG. 11 is an MA plot showing the results of RNAseq analysis in Experimental Example 5.

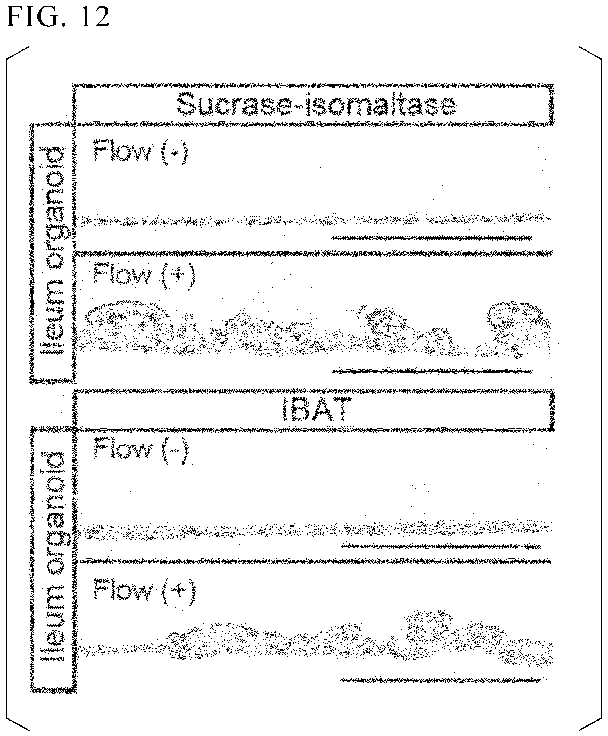

FIG. 12 is photographs showing the results of immunostaining in Experimental Example 6.

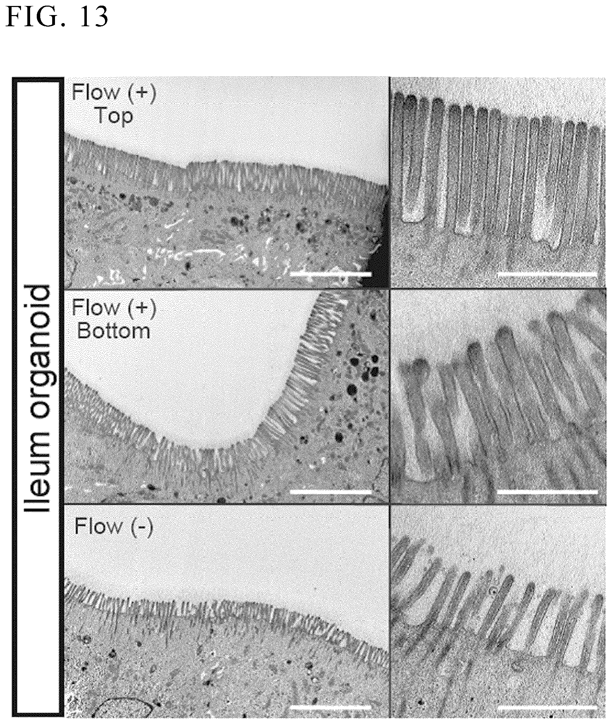

FIG. 13 is electron micrographs captured in Experimental Example 6.

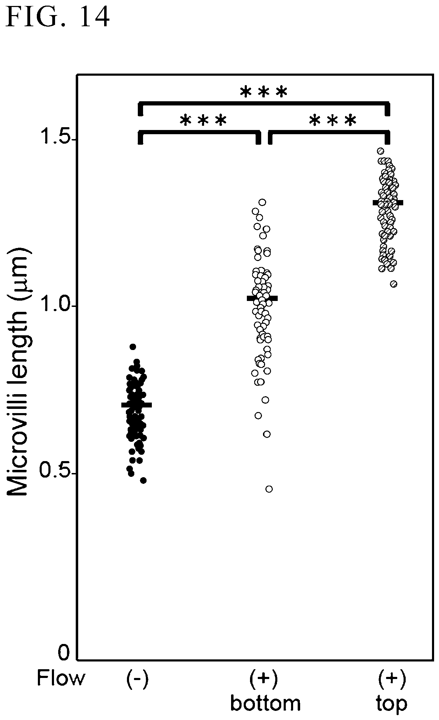

FIG. 14 is a graph showing the lengths of microvilli measured based on the electron micrographs of FIG. 13.

### [Description of Embodiments]

### [Method for producing two-dimensional small intestinal organoid having villus structure]

In an embodiment, the present invention provides a method for producing a two-dimensional small intestinal organoid having a villus structure, the method including a step 1 of culturing a cell derived from a small intestinal epithelium in an extracellular matrix to obtain a three-dimensional small intestinal organoid, a step 2 of dispersing the three-dimensional small intestinal organoid and monolayer culturing on the extracellular matrix to obtain a two-dimensional small intestinal organoid, and a step 3 of further culturing the two-dimensional small intestinal organoid while letting a culture medium of the two-dimensional small intestinal organoid to flow so that the two-dimensional small intestinal organoid forms a villus structure.

As described below in examples, according to the production method of the embodiment, it is possible to produce a two-dimensional small intestinal organoid having a villus structure in vitro. In addition, as described below in the examples, the inventors clarified that, in a two-dimensional small intestinal organoid obtained by the production method of the embodiment, a cell that configures the villus structure expresses molecules that are involved in an intestinal digestion and absorption action and exhibits a small intestinal function.

### <Step 1>

In the step 1, a cell derived from a small intestinal epithelium is cultured in an extracellular matrix to obtain a three-dimensional small intestinal organoid. The cell derived from a small intestinal epithelium may be a normal cell or a tumor cell.

In the present specification, the small intestine refers to an intestinal tract having a villus structure that provides functions such as nutrient absorption and includes the duodenum, the jejunum, and the ileum. The small intestine is an organ having a function of further breaking down food digested in the stomach and absorbing nutrients.

In addition, in the present specification, the epithelial cell includes a differentiated epithelial cell and an epithelial stem cell acquired from the epithelial tissue. The epithelial stem cell means a cell having a long-term self-replication function and a differentiation ability into an epithelial differentiated cell and means a stem cell derived from an epithelial tissue.

As a method for isolating a cell from a small intestinal epithelial tissue, a well-known method in the art is an exemplary example. For example, a crypt can be isolated by leaving a chelating agent and a tissue at a constant temperature. After the tissue is washed, an epithelial cell layer is peeled off from a submucosa with a glass slide and minced. After this, the tissue is left at a constant temperature in a liquid containing trypsin and, preferably, at least one of EDTA and EGTA, and an undigested tissue fragment and a crypt-derived single cell are separated using at least any one of a filter and a centrifuge. Instead of trypsin, a different proteolytic enzyme such as collagenase or dispase I may be used.

In the present specification, the three-dimensional organoid means a three-dimensional cell organization that has been self-organized by accumulating cells in a controlled space at a high density. In addition, the two-dimensional organoid is a planar cell organization that is obtained by dispersing the three-dimensional organoid and culturing the three-dimensional organoid in a monolayer.

A conventional three-dimensional small intestinal organoid had a spherical shape and a structure in which the villus structure protruded from the surface, but the intestinal lumen was inside the spherical shape. Therefore, in order to examine absorption via villi or the like using the three-dimensional small intestinal organoid, an additional experimental technique was required. In contrast, the two-dimensional small intestinal organoid that is obtained by the production method of the embodiment is a two-dimensional cell organization having the villus structure of the intestinal lumen on the culture upper surface and is thus suitable for in vitro studies of absorption and metabolism mechanisms via villous tissues imitating the original intestinal lumen, influences of drugs on villus structures, and the growth of intestinal bacteria and viruses.

The extracellular matrix (ECM) means a supramolecular structure that is present outside the cells of a living body. The extracellular matrix serves as a scaffold for cell growth. The extracellular matrix encompasses various polysaccharides, water, elastin, glycoproteins, and the like. As the glycoproteins, for example, collagen, entactin (nidogen), fibronectin, laminin, and the like are exemplary examples.

The extracellular matrix may be a commercially available extracellular matrix or may be prepared. As the commercially available extracellular matrix, for example, a basement membrane preparation derived from an Engelbreth-Holm-Swarm (EHS) mouse sarcoma cell (for example, MATRIGEL (registered trademark, Corning Incorporated)) is an exemplary example. Additionally, synthetic extracellular matrices such as extracellular matrix protein (Thermo Fisher Scientific) and ProNectin (Sigma) may also be used. In addition, a mixture of a natural extracellular matrix and a synthetic extracellular matrix may also be used.

As a method for preparing the extracellular matrix, for example, a method in which a connective tissue cell is used or the like is an exemplary example. More specifically, after extracellular matrix-producing cells, for example, fibroblasts, are cultured, these cells are removed and an extracellular matrix can be recovered.

As the extracellular matrix-producing cells, for example, chondrocytes that mainly produce collagen and proteoglycan, fibroblasts that mainly produce type IV collagen, laminin, interstitial procollagen, and fibronectin, colonic myofibroblasts that mainly produce collagen (types I, III, and V), chondroitin sulfate proteoglycan, hyaluronic acid, fibronectin, and tenascin-C, and the like are exemplary examples.

Small intestinal epithelium-derived cells can be embedded in the extracellular matrix by mixing the extracellular matrix with the small intestinal epithelium-derived cells and solidifying the mixture at 37°C. Subsequently, three-dimensional culture is performed by overlaying a culture medium on the extracellular matrix containing the cells, whereby a three-dimensional small intestinal organoid can be obtained.

As the culture medium for producing the three-dimensional small intestinal organoid, a culture medium obtained by adding at least one of the following components i) to v) to a basal culture medium is preferable. The culture medium may contain serum, but preferably contains no serum.
i) Wnt agonist
ii) At least one selected from the group consisting of insulin-like growth factor 1 (IGF1), fibroblast growth factor 2 (FGF2), epidermal growth factor (EGF), and epiregulin (EREG)
iii) Bone morphogenetic protein (BMP) inhibitor
iv) Transforming growth factor-β (TGF-β) inhibitor
v) γ-secretase inhibitor

The culture medium for producing the three-dimensional small intestinal organoid preferably contains at least one selected from the group consisting of IGF 1, FGF2, EGF, and epiregulin (EREG). Which of IGF1, FGF2, EGF, and epiregulin is contained can be selected as appropriate. The culture medium for producing the three-dimensional small intestinal organoid preferably contains IGF1 and epiregulin, FGF2 and epiregulin, or IGF1 and FGF2 and more preferably contains IGF1 and FGF2.

As the basal culture medium, any serum-free cell culture basal medium can be used. For example, a prescribed synthetic culture medium buffered to pH 7.2 or higher and pH 7.6 or lower with a carbonate-based buffer solution or the like is an exemplary example. More specifically, an advanced-Dulbecco's modified eagle culture medium: Ham's Nutrient Mixture F-12 (DMEM/F12) supplemented with glutamine, insulin, B27 supplement (Thermo Fisher Scientific) N-acetyl-L-cysteine (Fujifilm Wako Pure Chemical Corporation), penicillin, streptomycin, transferrin, or the like and the like are exemplary examples. In addition, an RPMI1640 culture medium, an advanced RPMI culture medium, or the like may be used instead of the DMEM/F12 culture medium.

### (Wnt agonist)

The Wnt agonist means a drug that activates T-cell factor (hereinafter, also referred to as TCF)/lymphoid enhancer factor (hereinafter, also referred to as LEF)-mediated transcription in cells. Therefore, the Wnt agonist is not limited to Wnt family proteins, but includes Wnt agonists that bind to a Frizzled receptor family member to be activated, intracellular β-catenin degradation inhibitors, and TCF/LEF activators. The Wnt agonist is preferably at least one selected from the group consisting of a Wnt protein, R-spondin, and a GSK-3β inhibitor.

The culture medium for producing the three-dimensional small intestinal organoid preferably contains the Wnt agonist. As the Wnt agonist, a complex of a Wnt protein and afamin, which is a stabilizer thereof, is more preferably contained, and a complex of a Wnt protein and afamin and R-spondin are still more preferably contained. When a complex of a Wnt protein and afamin and R-spondin are contained in the culture medium, the three-dimensional small intestinal organoid can be formed with a higher efficiency.

### <<Wnt protein>>

Where the Wnt protein is derived is not particularly limited, and it is possible to use Wnt proteins derived from various organisms. Among them, mammal-derived Wnt proteins are preferable. As the mammal, human beings, mice, rats, cows, pigs, rabbits, and the like are exemplary examples. As the Wnt proteins of mammals, Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, Wnt16, and the like are exemplary examples. In the culture medium for producing the three-dimensional small intestinal organoid, a plurality of the Wnt proteins may be used in combination.

As a method for preparing the Wnt protein, for example, a method in which the Wnt protein is prepared using a Wnt protein-expressing cell and the like are exemplary examples. In the Wnt protein-expressing cell, where the cell is derived (organism species, culture morphology, or the like) is not particularly limited and may be a cell that stably expresses the Wnt protein or may be a cell that transiently expresses the Wnt protein. As the Wnt protein-expressing cell, for example, an L cell that stably expresses mouse Wnt3a (ATCC CRL-2647), an L cell that stably expresses mouse Wnt5a (ATCC CRL-2814), and the like are exemplary examples. In addition, the Wnt protein-expressing cell can be made using a well-known gene recombination technique. That is, the Wnt protein-expressing cell can be made by inserting a DNA that encodes a desired Wnt protein into a well-known expression vector and introducing the obtained expression vector into an appropriate host cell. The nucleotide sequence of a gene that encodes the desired Wnt protein can be acquired from a well-known nucleotide sequence database such as GenBank (R).

The Wnt protein that is expressed by the Wnt protein-expressing cell may be a fragment of the Wnt protein or may be a Wnt protein containing an amino acid sequence other than the amino acid sequence of the Wnt protein as long as the Wnt protein has a Wnt activity. The amino acid sequence other than the amino acid sequence of the Wnt protein is not particularly limited, and examples thereof include an amino acid sequence of an affinity tag and the like. In addition, the amino acid sequence of the Wnt protein does not need to fully coincide with an amino acid sequence that can be acquired from a well-known database such as GenBank (R) and may be an amino acid sequence that is substantially the same as an amino acid sequence that can be acquired from a well-known database as long as the amino acid sequence has a Wnt activity.

As the amino acid sequence that is substantially the same as an amino acid sequence of the Wnt protein that can be acquired from a well-known database such as GenBank (R), for example, amino acid sequences that can be acquired from a well-known database in which one to several amino acids are deficient, substituted or added and the like are exemplary examples.

The amino acid sequence in which one to several amino acids are deficient, substituted, or added means that, for example, as many amino acids as can be made deficient, substituted, or added by a well-known mutant peptide making method such as a site-directed mutagenesis method (10 or less is preferable, 7 or less is more preferable, and 6 or less is still more preferable) are deficient, substituted or added.

In addition, as the amino acid sequence that is substantially the same, for example, an amino acid sequence of which the identity to an amino acid sequence that can be acquired from a well-known database is at least 80% or more, preferably at least 85% or more, more preferably at least 90% or more, still more preferably at least 92% or more, particularly preferably at least 95% or more, and most preferably at least 99% or more or the like is an exemplary example.

The concentration of the Wnt protein is preferably 50 ng/mL or more, more preferably 100 ng/mL or more and 10 µg/mL or less, still more preferably 200 ng/mL or more and 1 µg/mL or less, and particularly preferably 300 ng/mL or more and 1 µg/mL or less.

### <<R-spondin>>

As the R-spondin, an R-spondin family consisting of R-spondin 1, R-spondin 2, R-spondin 3, and R-spondin 4 is an exemplary example. The R-spondin family is a secretory protein and is known to be involved in the activation and control of the Wnt signaling pathway. In the cell culture medium according to the embodiment, a plurality of types of R-spondin may be used in combination.

The R-spondin may be a fragment of the R-spondin or may contain an amino acid sequence other than the amino acid sequence of the R-spondin as long as the R-spondin has an R-spondin activity.

### <<GSK-3β (glycogen synthase kinase-3β) inhibitor>>

As the GSK-3β inhibitor, for example, a FRAT family member that inhibits the interaction between CHIR-99021 (CAS number: 252917-06-9), CHIR-98014 (CAS number: 252935-94-7), lithium, kenpaullone (CAS number: 142273-20-9), 6-bromoindirubin-30-acetoxime, SB216763 (CAS number: 280744-09-4), SB415286 (CAS number: 264218-23-7), or GSK-3 and axin, a FRAT-derived peptide, and the like are exemplary examples.

### <<Afamin>>

Afamin means a glycoprotein belonging to the albumin family and is known to be present in blood or body fluids. In serum that is usually added to culture media, afamin derived from an animal from which the serum has been collected is contained. Since impurities other than afamin are contained in the serum, it is preferable to use afamin alone instead of using the serum.

Where the afamin that is contained in culture media is not particularly limited, and it is possible to use afamin derived from various organisms. Among them, mammal-derived afamin is preferable. As the mammal, the same mammals as described above are exemplary examples. The amino acid sequences of major mammalian afamin and the nucleotide sequences of genes that encode the amino acid sequences can be acquired from a well-known database such as GenBank (R). For example, in GenBank (R), the amino acid sequence of human afamin is registered as AAA21612, the nucleotide sequence of the gene that encodes the amino acid sequence is registered with an accession number of L32140, the amino acid sequence of bovine afamin is registered as DAA28569, and the nucleotide sequence of the gene that encodes the amino acid sequence is registered with an accession number of GJ060968.

The afamin that is contained in the culture medium may be natural afamin that is contained in serum or the like and purified by a well-known method or may be recombinant afamin. The recombinant afamin can be produced by appropriately using a well-known gene recombination technique.

As a method for producing the recombinant afamin, the recombinant afamin can be produced by, for example, inserting DNA that encodes afamin into a well-known expression vector, introducing the obtained expression vector into an appropriate host cell to express recombinant afamin, and purifying the recombinant afamin using a well-known purification method. The recombinant afamin may be affinity-tagged afamin. The affinity tag to be added is not particularly limited and can be appropriately selected from well-known affinity tags and used. As the affinity tag, an affinity tag that is recognized by a specific antibody is preferable, and examples thereof include a FLAG tab, an MYC tag, an HA tag, a V5 tag, and the like.

The above-described Wnt protein has strong hydrophobicity because a specific serine residue is modified with a fatty acid (palmitoleic acid). Therefore, the Wnt protein is easily aggregated or denatured in an aqueous solution, and thus it is widely known that it is extremely difficult to purify and store the Wnt protein.

Incidentally, it has been reported that modification of this specific serine residue with a fatty acid is essential for the physiological activity of the Wnt protein and is involved in binding to the Frizzled receptor family member.

In addition, it is known that the Wnt protein binds to afamin on a one-to-one basis to form a complex in an aqueous solution and is solubilized while maintaining a high physiological activity. The Wnt protein-afamin complex may be produced by a method in which a cell that expresses both the Wnt protein and afamin is cultured, and the Wnt protein-afamin complex may be produced by a method in which a Wnt protein-expressing cell and an afamin-expressing cell are co-cultured.

The concentration of afamin contained in the culture medium is not particularly limited, but is preferably 50 ng/mL or higher and 10 µg/mL or lower, more preferably 100 ng/mL or higher and 1 µg/mL or lower, and still more preferably 300 µg/mL or higher and 1 µg/mL or lower.

### (IGF1)

IGF1 is also called somatomedin C and is a factor that is secreted mainly in the liver by stimulation by growth hormone (GH). It is known that almost all cells in a human body (particularly, cells in muscles, bones, liver, kidney, nerve, skin, lung, and the like) are affected by IGF1. In addition to an insulin-like effect, IGF1 has a function of regulating the growth and development of cells (particularly nerve cells) and cellular DNA synthesis.

The concentration of IGF 1 contained in the culture medium is not particularly limited, but is preferably 5 ng/mL or higher and 1 µg/mL or lower, more preferably 10 ng/mL or higher and 1 µg/mL or lower, and still more preferably 50 ng/mL or higher and 500 ng/mL or lower.

### (FGF2)

FGF2 is a basic fibroblast growth factor, binds to a fibroblast growth factor receptor (FGFR), and has a function of the growth promotion of vascular endothelial cells and the organization into a tubular structure, that is, the promotion of the new birth of blood vessels. In addition, it is known that human FGF2 has 2 isoforms of a low-molecular-weight type (LWL) and a high-molecular-weight type (HWL). LWL is present mainly in cytoplasm and acts by autocrine, whereas HWL is present in nuclei and exhibits activity by an intracrine mechanism that acts in cells.

The concentration of FGF2 contained in the culture medium is not particularly limited, but is preferably 5 ng/mL or higher and 1 µg/mL or lower, more preferably 10 ng/mL or higher and 1 µg/mL or lower, and still more preferably 50 ng/mL or higher and 500 ng/mL or lower.

### (EGF)

EGF is a potent mitogenic factor for various cultured ectodermal cells and mesodermal cells and has a significant influence on the differentiation of some fibroblasts into specific cells. An EGF precursor is present as a membrane-bound molecule that is proteolytically cleaved to produce 53-amino acid peptide hormone that stimulates cells.

The concentration of EGF contained in the culture medium is preferably 5 ng/mL or higher and 500 ng/mL or lower, more preferably 10 ng/mL or higher and 400 ng/mL or lower, and still more preferably 50 ng/mL or higher and 200 ng/mL or lower.

### (EREG)

EREG is an EGF-like growth factor that specifically binds to ErbB 1 and ErbB4 among tyrosine kinase (ErbB) family receptors (ErbB 1 to 4). It is known that EREG stimulates the growth of keratinocytes, hepatocytes, fibroblasts, and vascular endothelial cells. In addition, EREG is expressed mainly in malignant tumors in bladder, lung, kidney, colon, and the like, placenta, and peripheral blood leukocytes.

The concentration of EREG contained in the culture medium is not particularly limited, but is preferably 5 ng/mL or higher and 1 µg/mL or lower, more preferably 10 ng/mL or higher and 1 µg/mL or lower, and still more preferably 50 ng/mL or higher and 500 ng/mL or lower.

### (BMP inhibitor)

BMP binds as a dimeric ligand to a receptor complex consisting of two different receptors serine/threonine kinases: type I and type II receptors. The type II receptor phosphorylates the type I receptor, and, as a result, this receptor kinase is activated. This type I receptor subsequently phosphorylates a specific receptor substrate (SMAD), and, as a result, a transcriptional activity is guided via a signaling pathway. Generally, a BMP inhibitor is an agent that, for example, blocks or inhibits the binding of a BMP molecule to a BMP receptor and a drug that binds to the BMP molecule to form a complex that neutralizes the BMP activity. In addition, the BMP inhibitor is an agent that, for example, binds to a BMP receptor and blocks or inhibits the binding of a BMP molecule to the receptor and a drug that acts as an antagonist or inverse agonist.

The BMP inhibitor has an inhibitory activity of preferably 50% or more, more preferably 70% or more, still more preferably 80% or more, and particularly preferably 90% or more compared to the BMP activity level in the absence of this inhibitor.

The BMP inhibitor is preferably a naturally occurring BMP binding protein, and examples thereof include chordin-like proteins such as noggin, gremlin, chordin, and chordin domains; follistatin-related proteins such as follistatin and follistatin domain; DAN-like proteins such as DAN and DAN cysteine-knot domain; sclerostin/SOST, decorin, α-2 macroglobulin, and the like.

The BMP inhibitor contained in the culture medium is, particularly, preferably a chordin-like protein or a DAN-like protein and more preferably a chordin-like protein. The chordin-like protein is preferably noggin. The chordin-like protein or the DAN-like protein is a diffusible protein and is capable of binding to a BMP molecule with various affinities and inhibiting the access of the BMP molecule to a signaling receptor. In a case where an epithelial stem cell is cultured, the loss of the stem cell can be prevented by adding these BMP inhibitors to the cell culture medium.

The concentration of the BMP inhibitor contained in the culture medium is preferably 10 ng/mL or higher and 100 ng/mL or lower, more preferably 20 ng/mL or higher and 100 ng/mL or lower, and still more preferably 50 ng/mL or higher and 100 ng/mL or lower.

### (TGF-β inhibitor)

TGF-β is a type of growth factor and is produced in almost all cells of kidney, bone marrow, platelets, and the like. As TGF-β, there are five subtypes (β1 to β5). In addition, it is known that TGF-β promotes the growth of osteoblasts and the synthesis and growth of connective tissues such as collagen and acts to suppress the growth of epithelial cells and osteoclasts. Generally, a TGF-β inhibitor is an agent that, for example, blocks or inhibits the binding of TGF-β to a TGF-β receptor and a drug that binds to TGF-β to form a complex that neutralizes the TGF-β activity. In addition, the TGF-β inhibitor is an agent that, for example, binds to a TGF-β receptor and blocks or inhibits the binding of TGF-β to the receptor and a drug that acts as an antagonist or inverse agonist.

As the TGF-β inhibitor, for example, A83-01 (CAS number: 909910-43-6), ALK5 inhibitor I (3-(pyridin-2-yl)-4-(4-quinonyl)-1H-pyrazole), LDN193189 (CAS number: 1062368-24-4), SB-431542 (CAS number: 301836-41-9), SB-505124 (CAS number: 694433-59-5), SD-208 (CAS number: 627536-09-8), SB-525334 (CAS number: 356559-20-1), LY364947 (CAS number: 396129-53-6), LY2157299 (CAS number: 700874-72-2), TGF-β RI kinase inhibitor II 616452 (CAS Number: 446859-33-2), TGF-β RI kinase inhibitor III 616453 (CAS Number: 356559-13-2), TGF-β RI kinase inhibitor IX 616463 (4-((4-((2,6-dimethylpyridin-3-yl)oxy)pyridin-2-yl)amino)benzenesulfonamide), TGF-β RI kinase inhibitor VII 616458 (CAS Number: 666729-57-3), TGF-β RI kinase inhibitor VIII 616459 (CAS number: 356559-20-1), AP12009 (TGF-β2 antisense compound "Trabedersen"), belagen pumatucel-L (TGF-β2 antisense gene-modified allogeneic tumor cell vaccine), CAT-152 (glaucoma-lerdelimumab (anti-TGF-β-2 monoclonal antibody)), CAT-192 (metelimumab (human IgG4 monoclonal antibody that neutralizes TGFβ1), GC-1008 (anti-TGF-β monoclonal antibody), and the like are exemplary examples. As the TGF-β inhibitor, among these, A83-01 is preferable.

The concentration of the TGF-β inhibitor contained in the culture medium is preferably 100 nM or higher and 10 µM or lower, more preferably 500 nM or higher and 5 µM or lower, and still more preferably 500 nM or higher and 2 µM or lower.

### (γ-secretase inhibitor)

As γ-secretase, for example, BMS299897 (CAS number: 290315-45-6), DAPT (CAS number: 208255-80-5), DBZ (CAS number: 209984-56-5), JLK6 (CAS number: 62252-26-0), L-685458 (CAS number: 292632-98-5), LY411575 (CAS number: 209984-57-6), and the like are exemplary examples. Among these, LY411575 is preferable.

The concentration of the γ-secretase inhibitor contained in the culture medium is preferably 1 nM or higher and 10 µM or lower and more preferably 100 nM or higher and 1 µM or lower.

### (Other components)

The culture medium may further contain a Rho-kinase (Rock) inhibitor. As the Rock inhibitor, for example, Y-27632 (CAS number: 129830-38-2), fasudil (HA1077) (CAS number: 103745-39-7), H-1152 (CAS number: 871543-07-6), and the like are exemplary examples. In a case where Y-27632 is used as the Rock inhibitor, it is preferable to add Y-27632 during the first two days of the culture of stem cells dispersed in a single cell. Y-27632 contained in the culture medium is preferably about 10 µM.

To the culture medium, gastrin (or an appropriate substitute such as Leu15-gastrin) may be further added. The concentration of gastrin or the appropriate substitute contained in the culture medium may be, for example, 1 ng/mL or higher and 10 µg/mL, for example, 1 ng/mL or higher and 1 µg/mL or lower or, for example, 5 ng/mL or higher and 100 ng/mL or lower.

The culture medium may further contain at least one amino acid. As the amino acid, for example, L-alanine, L-arginine, L-asparagine, L-aspartic acid, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, L-valine, combinations thereof, and the like are exemplary examples. The concentration of L-glutamine contained in the culture medium is 0.05 g/L or higher and 1 g/L or lower (usually 0.1 g/L or higher and 0.75 g/L or lower). Other amino acids contained in the medium are 0.001 g/L or higher and 1 g/L (usually 0.01 g/L or higher and 0.15 g/L or lower). The amino acid may be synthetic amino acid.

The culture medium may further contain at least one vitamin. As the vitamin, for example, thiamine (vitamin B1), riboflavin (vitamin B2), niacin (vitamin B3), calcium D-pantothenate (vitamin B5), pyridoxal/pyridoxamine/pyridoxine (vitamin B6), folic acid (vitamin B9), cyanocobalamin (vitamin B12), ascorbic acid (vitamin C), calciferol (vitamin D2), DL-α tocopherol (vitamin E), biotin (vitamin H), menadione (vitamin K), and the like are exemplary examples.

The culture medium may further contain at least one inorganic salt. The Inorganic salt is contained to help maintain the osmotic balance of cells and to help regulate the membrane potential. Specific examples of the inorganic salt include salts of calcium, copper, iron, magnesium, potassium, sodium, and zinc. The salt is usually used in a form of chloride, phosphate, sulfate, nitrate, or bicarbonate. More specific examples of the salt include CaCL₂, CuSO₄-5H₂O, Fe(NO₃)-9H₂O, FeSO₄-7H₂O, MgCl, MgSO₄, KCl, NaHCO₃, NaCl, Na₂HPO₄, Na₂HPO₄-H₂O, ZnSO₄-7H₂O, and the like.

The culture medium may further contain at least one sugar that can serve as a carbon energy source. As the sugar, for example, glucose, galactose, maltose, fructose, and the like are exemplary examples. Among these, glucose is preferable, and D-glucose (dextrose) is particularly preferable. The concentration of the sugar contained in the culture medium is preferably 1 g/L or higher and 10 g/L.

The culture medium may further contain at least one trace element. As the trace element, for example, barium, bromium, cobalt, iodine, manganese, chromium, copper, nickel, selenium, vanadium, titanium, germanium, molybdenum, silicon, iron, fluorine, silver, rubidium, tin, zirconium, cadmium, zinc, aluminum, ions thereof, and the like are exemplary examples.

The culture medium may further contain at least one additional drug. As such a drug, nutrients or growth factors reported to improve stem cell culture, for example, cholesterol, transferrin, albumin, insulin, progesterone, putrescine, selenite, and the like are exemplary examples.

### <Step 2>

In the step 2, the three-dimensional small intestinal organoid obtained in step 1 is dispersed and subjected to monolayer culturing on an extracellular matrix to obtain a two-dimensional small intestinal organoid. In the present step, the three-dimensional small intestinal organoid is dispersed in one to several cells. As a method for dispersing the three-dimensional small intestinal organoid, a physical method, an enzymatic treatment method, and the like are exemplary examples, and the enzymatic treatment method is preferable from the viewpoint of not damaging cells. As an enzyme that is used in the enzymatic treatment method, trypsin, collagenase, dispase I, and the like are exemplary examples.

Subsequently, the dispersed cells are seeded onto an extracellular matrix. For example, the surface of a cell culture insert may be coated with an extracellular matrix, and the dispersed cells may be seeded onto this extracellular matrix. The seeded cells are capable of adhering to the extracellular matrix by interacting with the surface structure of the extracellular matrix, for example, interacting with integrins. The extracellular matrix is the same as described above.

In addition, it is also possible to seed and culture cells such as fibroblasts and endothelial cells on a cell culture insert and seed the dispersed cells on the cultured cells.

As the cell culture insert, an insert having a membrane on the bottom surface can be used. The material of the membrane may be a material that can be used for cell culture such as polyester (PET), polycarbonate, or collagen-coated polytetrafluoroethylene (PTFE). In addition, the pore sizes in the membrane are not particularly limited and may be 0.1 to 10 µm, for example, 0.1 to 1.0 µm. Even when the pore sizes are outside the above-described range, the membrane can still be used.

Subsequently, after the seeding of the cells, before the cells are dried, a culture medium is added and the cells are subjected to monolayer culturing. Thereby, a two-dimensional organoid is obtained. As the culture medium, it is possible to use the same culture medium as the culture medium for producing the three-dimensional small intestinal organoid described above.

### <Step 3>

Subsequently, in the step 3, the two-dimensional small intestinal organoid obtained in the step 2 is further cultured while causing the culture medium of the two-dimensional small intestinal organoid to flow. As a result, the two-dimensional small intestinal organoid forms a villus structure. The flow of the culture medium is preferably initiated after the two-dimensional organoid becomes confluent.

The flow of the culture medium mimics the flow of an intestinal fluid in the small intestine of a living body. Therefore, the flow of the culture medium preferably imparts the same degree of stimulation to the two-dimensional small intestinal organoid as stimulation that the small intestinal epithelium receives by the flow of an intestinal fluid.

For example, the culture medium can be made to flow by shaking a cell culture vessel on a shaker. The shaker may be a rotary shaker. As described below in the examples, when the rate of the flow of the culture medium is set to about 150 rpm, it is possible to promote the formation of a villus structure in two-dimensional small intestinal organoid.

### [Two-dimensional small intestinal organoid having villus structure]

In an embodiment, the present invention provides a two-dimensional small intestinal organoid having a villus structure. Conventionally, it was not possible to produce a two-dimensional small intestinal organoid having a villus structure. In contrast, as described below in the examples, the inventors clarified that a two-dimensional small intestinal organoid having a villus structure can be produced in vitro by the above-described production method. The two-dimensional small intestinal organoid of the embodiment may be a two-dimensional small intestinal organoid obtained by the above-described production method.

The use of the two-dimensional small intestinal organoid of the embodiment makes it possible to investigate the mechanism relating to absorption or the like in the intestinal tract. In addition, the use of the two-dimensional small intestinal organoid of the embodiment makes it possible to perform the screening or the like of therapeutic agents for a small intestinal dysfunction.

The two-dimensional small intestinal organoid of the embodiment may be produced from a cell derived from a normal small intestine, may be produced from a cell derived from a patient with a small intestinal dysfunction, or may be produced from a cell derived from a patient with a small intestinal disease. As the cell derived from a normal small intestine, it is possible to use a normal cell or the like collected from the normal duodenum or ileum tissue of a patient with a gastric tumor or colon tumor. The two-dimensional small intestinal organoid produced from a cell derived from a patient with small intestinal dysfunction can be used as a small intestinal dysfunction model. The two-dimensional small intestinal organoid produced from a cell derived from patient with a small intestinal disease can be used as a small intestinal disease model. For example, the two-dimensional small intestinal organoid produced from a cell derived from an inflammatory bowel disease can be used as an inflammatory bowel disease model.

### [Method for screening absorption controller of nutrient or drug in small intestine]

In an embodiment, the present invention provides a method for screening an absorption controller of a nutrient or a drug in a small intestine, the method including a step of measuring the absorption amount of a nutrient or a drug by a two-dimensional small intestinal organoid having a villus structure in the presence of a test substance, in which a case where the absorption amount is significantly changed compared with that in the absence of the test substance indicates that the test substance is an absorption controller of a nutrient or a drug in a small intestine.

The test substance is not particularly limited, and examples thereof include a natural compound library, a synthetic compound library, an existing drug library, a metabolite library, and the like.

As the nutrient, lipids, sugars, amino acids, and the like are exemplary examples. A method for measuring the absorption amount of the nutrient may be selected as appropriate depending on a nutrient to be measured. For example, the absorption amount of a nutrient can be quantified by absorbing a fluorescent-labeled nutrient in the two-dimensional small intestinal organoid and measuring the fluorescence intensity based on a fluorescence micrograph thereof. In addition, the drug is not particularly limited and may be, for example, a medical drug or the like.

The absorption controller of a nutrient or a drug in the small intestine may be a substance that promotes the absorption of a nutrient or a drug in the small intestine or a substance that suppresses the absorption of a nutrient or a drug in the small intestine.

The substance that promotes the absorption of a nutrient in the small intestine can be used as, for example, a therapeutic agent for a small intestinal dysfunction. In addition, the substance that suppresses the absorption of a nutrient in the small intestine can be used as, for example, an anti-obesity drug.

In a conventional three-dimensional small intestinal organoid, the villus structure protruded from the surface but was a spherical structure, and cells that configured the intestinal lumen were present in the lumen of the organoid. Therefore, an additional experimental technique was required to examine phenomena occurring in the intestinal lumen, digestion and absorption, culture of intestinal microorganisms, and the like using a three-dimensional small intestinal organoid.

In contrast, according to the screening method of the embodiment, since the two-dimensional small intestinal organoid is a two-dimensional cell organization having a villus structure that imitates the intestinal lumen on the culture upper surface, the method is suitable for in vitro analysis of phenomena occurring in the intestinal lumen, such as absorption mechanisms via villous tissues.

### [Method for screening formation controller of villus structure in small intestine]

In an embodiment, the present invention provides a method for screening a formation controller of a villus structure in a small intestine, the method including a step of producing a two-dimensional small intestinal organoid having a villus structure in the presence of a test substance and a step of evaluating the morphology of the villus structure of the produced two-dimensional small intestinal organoid, in which a case where the morphology is significantly changed compared with that in the absence of the test substance indicates that the test substance is a formation controller of a villus structure in a small intestine.

As the test substance, the same test substance as described above can be used. In addition, the step of producing a two-dimensional small intestinal organoid having a villus structure is the same as the above-described method for producing a two-dimensional small intestinal organoid.

The test substance may be added to a culture medium throughout the entire step of producing a two-dimensional small intestinal organoid or may be added to a culture medium during a part of the step of producing a two-dimensional small intestinal organoid.

As the morphology of the villus structure, for example, the density of the villus structure, the height of the villus structure, the density of microvilli, the lengths of microvilli, and the like are exemplary examples. The villus structure can be observed with an optical microscope or the like. In addition, the microvilli can be observed with an electron microscope or the like.

The formation controller of a villus structure may be a substance that promotes the formation of a villus structure or a substance that suppresses the formation of a villus structure.

The substance that promotes the formation of a villus structure can be used as, for example, a therapeutic agent for a small intestinal dysfunction. In addition, the substance that suppresses the formation of a villus structure can be used as, for example, an anti-obesity drug.

### [Method for screening regulator of digestion, absorption, or metabolism of target substance in small intestine]

In an embodiment, the present invention provides a method for screening a regulator of digestion, absorption, or metabolism of a target substance in a small intestine, the method including a step of producing a two-dimensional small intestinal organoid having a villus structure in the presence of the test substance and a step of measuring digestion, absorption, or metabolism of the target substance in the produced two-dimensional small intestinal organoid to obtain a measurement value, in which a case where the measurement value is significantly changed compared with that in the absence of the test substance indicates that the test substance is a regulator of digestion, absorption, or metabolism of the target substance in a small intestine.

As the test substance, the same test substance as described above can be used. In addition, the step of producing a two-dimensional small intestinal organoid having a villus structure is the same as the above-described method for producing a two-dimensional small intestinal organoid.

The test substance may be added to a culture medium throughout the entire step of producing a two-dimensional small intestinal organoid or may be added to a culture medium during a part of the step of producing a two-dimensional small intestinal organoid.

The target substance is not particularly limited, and examples thereof include a nutrient, a drug, and the like. As the nutrient and the drug, the same nutrient and drug as described above are exemplary examples. A method for measuring the digestion, absorption, or metabolism of the target substance may be selected as appropriate depending on the target substance. Examples thereof include liquid chromatography (LC), a liquid chromatography-mass spectrometer (LC-MS), and the like.

The regulator of the digestion, absorption, or metabolism of the target substance can be used as, for example, a therapeutic agent for a small intestinal dysfunction.

### [Method for screening substance that regulates growth of intestinal bacterium and/or virus in small intestine]

In an embodiment, the present invention provides a method for screening a substance that regulates growth of an intestinal bacterium and/or a virus in a small intestine (growth regulator), the method including a step of measuring growth of an intestinal bacterium and/or a virus in a two-dimensional small intestinal organoid having a villus structure in the presence of a test substance, in which a case where the growth of the intestinal bacterium and/or virus is significantly changed compared with that in the absence of the test substance indicates that the test substance is a substance that regulates growth of an intestinal bacterium and/or a virus in a small intestine.

A method for measuring the growth of an intestinal bacterium and/or a virus may be selected as appropriate. Examples thereof include a colony counting method, a plaque assay method, a quantitative PCR method, and the like.

The substance that regulates the growth of an intestinal bacterium and/or a virus can be used as a function regulator of the small intestine and also can be used as a regulator of an immune function by a change in intestinal flora, a virus infection controlling agents (antiviral agent), and the like.

### [Transplant material]

In an embodiment, the present invention provides a transplant material containing a small intestinal organoid, the transplant material that is used for transplantation into a large intestine of a living body to cause an intestinal fluid to flow so that a tissue having a small intestinal function is formed in the large intestine.

As described below in the examples, when a small intestinal organoid was transplanted in a large intestine of a living body, and an intestinal fluid was caused to flow on the organoid, it was possible to form a tissue having a small intestinal function in the large intestine.

In addition, a graft having a small intestinal function, which was made by transplanting a small intestinal organoid into the large intestine of a rat short bowel syndrome model, was capable of significantly improving the symptom of a small intestinal dysfunction. In contrast, in a case where a graft made by transplanting a colon organoid into the large intestine of a rat short bowel syndrome model was used, a rat died. Therefore, the transplantation of the transplant material of the embodiment into large intestines is useful as a treatment method of small intestinal dysfunction.

The small intestinal organoid to be transplanted may be a three-dimensional organoid or a dispersion of a three-dimensional organoid. Prior to the transplantation of a small intestine into a large intestine, it is preferable to peel off and remove the large intestine epithelium. The large intestine epithelium can be peeled off using, for example, EDTA.

### [Other embodiments]

In an embodiment, the present invention provides a treatment method of a small intestinal dysfunction, the treatment method including transplanting an effective amount of a small intestinal organoid into a large intestine of a patient in need of treatment so as to cause an intestinal fluid flow to form a tissue having a small intestinal function in the large intestine. In the treatment method of the embodiment, the small intestinal organoid is the same as that described above.

### [Examples]

Next, the present invention will be described in more detail by showing examples, but the present invention is not limited to the following examples.

### [Method]

### (Animal)

All animal experiments were performed with the approvals of Laboratory Animal Center, Keio University School of Medicine and the Institutional Review Board of Nippon Veterinary and Life Science University.

NOD. Cg-Prkdc^{scid} Il2rg^{tm1Sug}/Jic(NOG) mice (male, 6 to 12 weeks old) were obtained from Central Institute for Experimental Animals (CIEA). In addition, Lewis (LEW/Crl) rats (male, 7 to 10 weeks old) were obtained from Charles River Laboratories.

The mice were bred in a specific pathogen-free environment, and the rats were bred in a usual rat cage with wood chips laid. All animals were handled according to institutional guidelines and regulations.

### (Establishment and culture of human and rat intestinal organoids)

All experiments relating to human tissues were performed with the approvals of Keio University School of Medicine and the Ethics Committee of the University of Tokyo.

The human intestinal organoids were established from patients with a written informed consent.

The rat intestinal samples were collected from luciferase-expressing LEW transgenic rats (LEW-Tg ((ROSA)26Sor-luc)11Jmsk; luc-LEW Tg).

Crypts derived from rat ilea and colons were isolated by a standard method. The isolated crypts were mixed with 25 µL of MATRIGEL (registered trademark, Corning Incorporated) and seeded to a 48-well plate. After MATRIGEL (registered trademark) was polymerized, 250 µL of a culture medium was overlaid on the embedded crypts. The composition of the culture medium is shown in Table 1 below. The culture medium was changed every 2 to 3 days. In addition, the obtained intestinal organoids were passaged every 5 to 7 days by being treated with TrypLE Express (Thermo Fisher Scientific) and then gently pipetted.

**[Table 1]**

| Composition of culture medium |
|---|
| Advanced DMEM/F-12 culture medium |
| 10 mM HEPES |
| 2 mM GlutaMAX |
| 100 U/mL penicillin/100 µg/mL streptomycin (Thermo Fisher Scientific) |
| 1 mM N-acetylcysteine (Fujifilm Wako Pure Chemical Corporation) |
| 1 × B27 (Thermo Fisher Scientific) |
| 10 nM gastrin I (Sigma-Aldrich Inc.) |
| 25% Afamin-Wnt-3A serum-free conditioned medium |
| 50 ng/mL mouse recombinant EGF (Thermo Fisher Scientific) |
| 100 ng/mL mouse recombinant noggin (PeproTech, Inc.) |
| 10%R-spondin1 conditioned medium |
| 500 nMA83-01 (R&D Systems, Inc.) |
| 10 µM Y-27632 (Fujifilm Wako Pure Chemical Corporation) |
| 50 µg/mL-1 human FGF-2 (PeproTech, Inc.) |
| 100 µg/mL human IGF-1 (BioLegend, Inc.) |

### (Xenotransplantation of human intestinal organoid)

Two independent human ileal organoid lines were each labeled with a green fluorescent protein (GFP) or a red fluorescent protein (RFP) by introducing a GFP-puro PiggyBac vector (System Biosciences, LLC) or an RFP-puro PiggyBac vector (System Biosciences, LLC) by electroporation. In addition, a GFP-labeled human colon organoid was also used.

The human colon organoid or the human ileal organoid was passaged and, furthermore, expansion-cultured for 3 or 4 days. Subsequently, a NOG mouse was put under anesthesia, and the large intestine epithelium was partially peeled off with EDTA. Subsequently, the human colon organoid or the human ileal organoid was transplanted into the large intestine of the NOG mouse by transanal injection.

### (Monolayer culturing of human intestinal organoid)

A human duodenal organoid, a human ileal organoid, and a human colon organoid were expansion-cultured in MATRIGEL (registered trademark) for 5 to 7 days. Subsequently, each organoid was dissociated and seeded onto a ThinCert 24-well plate having a pore size of 0.4 µm coated with 20% MATRIGEL (registered trademark) (Greiner Bio-One International GmbH). 2×10⁵ cells were seeded into each ThinCert insert and cultured in an expansion culture medium.

Subsequently, after at least 3 days from the seeding and the cells became confluent, the culture media were caused to flow at 150 rpm using a rotary shaker (Thermo Fisher Scientific). The two-dimensional organoids were analyzed 4 days from the initiation of the flow of the culture media.

### [Experimental Example 1]

### (Xenotransplantation of human ileal organoid or human colon organoid)

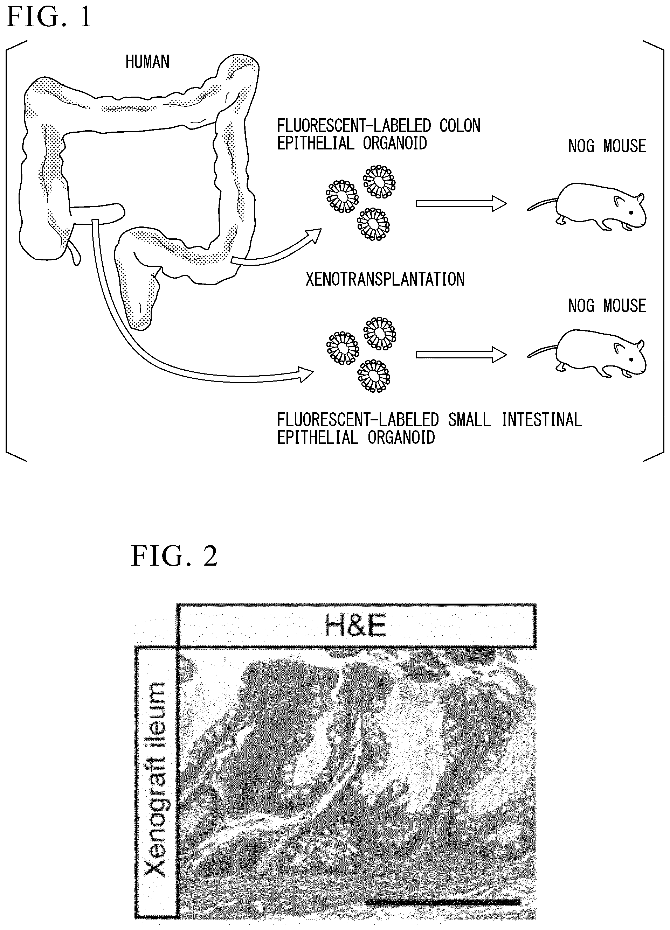

FIG. 1 is a schematic view showing a procedure for transplanting human ileal organoids or human colon organoids into the large intestines of immunodeficient mice (NOG mice). As shown in FIG. 1, human small intestinal organoids were xeno-transplanted onto the colorectal surfaces of the NOG mice, and whether or not a human small intestinal epithelium had a capability of remodeling organs was examined. In addition, for comparison, a human colon organoid was xeno-transplanted into a NOG mouse.

As a result, the human colon organoid formed a crypt structure histologically comparable to the crypt structure of the human colon as a xenograft. In contrast, the xeno-transplanted human ileal organoid constructed a neovillus structure reminiscent of a small intestinal epithelium.

FIG. 2 is a micrograph showing the result of hematoxylin and eosin staining of a tissue section of a human ileal organoid graft. The scale bar indicates 200 µm.

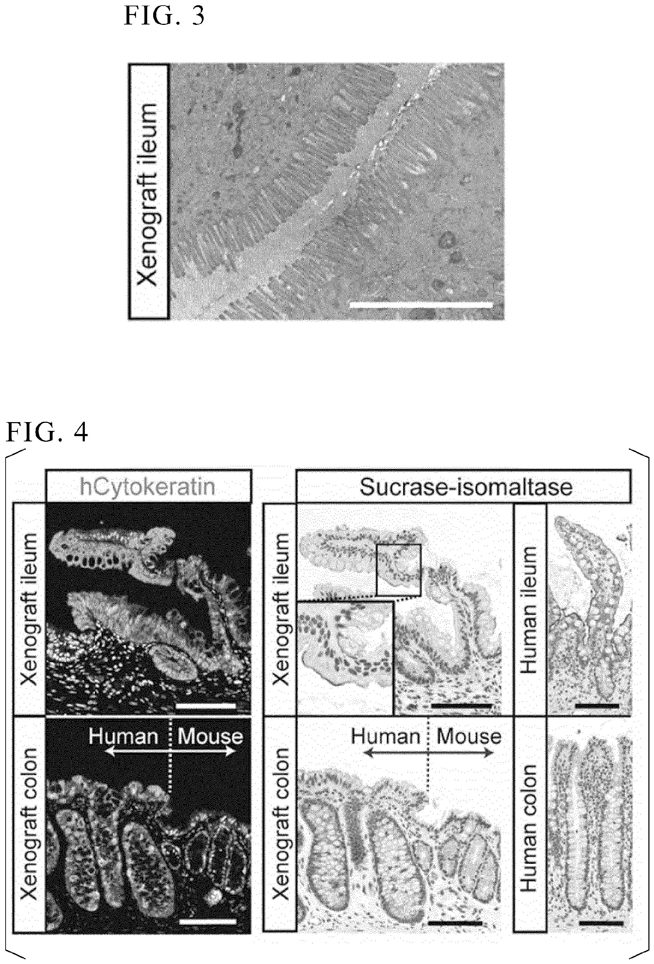

In addition, FIG. 3 is a transmission electron micrograph of a tissue section of a human ileal organoid graft. The scale bar is 4 µm. As shown in FIG. 3, it was clarified that the human ileal organoid graft formed a mature microvillus structure.

### [Experimental Example 2]

### (Examination of expression of marker protein in graft)

Expression of marker proteins in the human ileal organoid graft and a human colon organoid graft obtained in Experimental Example 1 was examined.

FIG. 4 is photographs showing the results of detecting the expression of human cytokeratin and sucrase-isomaltase in human ileal organoid grafts and human colon organoid grafts. The scale bar indicates 100 µm. Cytokeratin is a marker of an epithelial tissue. In addition, sucrase-isomaltase is a small intestine-specific marker.

The upper left part shows the result of detecting the expression of the human cytokeratin in the human ileal organoid graft, and the lower left part shows the result of detecting the expression of the human cytokeratin in the human colon organoid graft.

In addition, the upper middle part shows the result of detecting the expression of the sucrase-isomaltase in the human ileal organoid graft, and the upper right part shows the result of detecting the expression of the sucrase-isomaltase in a human ileum tissue for reference.

In addition, the lower middle part shows the result of detecting the expression of the sucrase-isomaltase in the human colon organoid graft, and the lower right part shows the result of detecting the expression of the sucrase-isomaltase in a human colon tissue for reference.

As a result, it was clarified that the sucrase-isomaltase was expressed only in the human ileal organoid graft and expression was not recognized in the human colon organoid graft. The above-described results demonstrate that the human small intestinal epithelial organoid has a capability of reconstructing a human small intestinal epithelial structure in the mouse intestinal tract.

### [Experimental Example 3]

### (Analysis of chyle vessel-like structure in graft)

The structures and functions of the human ileal organoid graft and the human colon organoid graft obtained in Experimental Example 1 were analyzed.

FIG. 5 is photographs showing the results of detecting the expression of a lymphatic vessel endothelial hyaluronan receptor 1 (Lyve-1) in a human ileal organoid graft and a human colon organoid graft. The scale bar indicates 200 µm.

As a result, it was clarified that, in the mouse colon, the human ileal organoid graft formed a small intestine-specific structure called a Lyve-1-positive chyle vessel in a crypt-villus-like structure. In contrast, in the human colon organoid graft, a lymphatic vessel was present mainly in a submucosa, and the formation of a chyle vessel was not recognized.

Subsequently, NBD-cholesterol, which is fluorescent-labeled cholesterol, was administered to a mouse having a graft, and whether or not a chyle vessel-like structure had a function of a lymphatic vessel that absorbs lipids was examined.

The left part of FIG. 6 is a photograph showing the result of detecting the presence of the NBD-cholesterol and the Lyve-1 in the human ileal organoid graft. The scale bar indicates 100 µm. The right part of FIG. 6 is a photograph showing the result of detecting the presence of RFP and Lyve-1, which shows the presence of a human ileal organoid graft, in the same visual field as in the left part of FIG. 6. The scale bar indicates 100 µm.

The upper part of FIG. 7 is a photograph showing the result of detecting RFP, which shows the presence of the human ileal organoid graft, in the boundary between a recipient's large intestine and the human ileal organoid graft. The scale bar indicates 50 µm. The lower part of FIG. 7 is a photograph showing the result of detecting NBD-cholesterol in the same visual field as in the upper part of FIG. 7. The scale bar indicates 50 µm.

As a result, it was clarified that the chyle vessel-like structure formed by the human ileal organoid graft has a function of absorbing lipids.

The above-described results demonstrate that the xeno-transplanted human ileal organoid is capable of reconstructing a small intestinal epithelium having an absorption-related mechanism.

### [Experimental Example 4]

### (Examination of influence of flow of culture medium on formation of villus structure by human small intestinal organoid)

An influence of the flow of a culture medium on the formation of a villus structure by a human small intestinal organoid was examined. FIG. 8 is a schematic view showing the outline of an experiment. First, a human colon organoid and a human small intestinal organoid formed two-dimensional organoids having a monolayer of polarized cells. Subsequently, a culture plate was placed on a rotator, and a constant flow was generated in a culture medium on the cells.

The two-dimensional organoids were formed as described below. A human duodenal organoid and a human colon organoid were expansion-cultured in MATRIGEL (registered trademark) for 5 to 7 days. Subsequently, each organoid was dissociated and seeded onto a ThinCert 24-well plate having a pore size of 0.4 µm coated with 20% MATRIGEL (registered trademark) (Greiner Bio-One International GmbH). 2×10⁵ cells were seeded into each ThinCert insert and cultured in an expansion culture medium.

Subsequently, after 3 days from the seeding and the cells became confluent, the culture media were caused to flow at 150 rpm using a rotary shaker (Thermo Fisher Scientific). The two-dimensional organoids were analyzed 4 days from the initiation of the flow of the culture media.

FIG. 9 is hematoxylin and eosin-stained images and micrographs of sections made by culturing a two-dimensionally cultured human duodenal organoid and a two-dimensionally cultured human colon organoid for 4 days in the presence and absence of the flow of a culture medium and fixing and embedding the organoids in paraffin. The scale bar is 200 µm. In FIG. 9, "Flow (-)" indicates that the organoids were cultured in the absence of the flow of the culture medium, and "Flow (+)" indicates that the organoids were cultured in the presence of the flow of the culture medium.

The top left part of FIG. 9 is the result of the human duodenal organoid cultured in the absence of the flow of the culture medium. The hematoxylin and eosin-stained images of the cross sections of the two-dimensional organoids are shown in the upper part, and the micrographs are shown in the lower part.

The bottom left part of FIG. 9 is the result of the human duodenal organoid cultured in the presence of the flow of the culture medium. The hematoxylin and eosin-stained images of the cross sections of the two-dimensional organoids are shown in the upper part, and the micrographs are shown in the lower part.

The top right part of FIG. 9 is the result of the human colon organoid cultured in the absence of the flow of the culture medium. The hematoxylin and eosin-stained images of the cross sections of the two-dimensional organoids are shown in the upper part, and the micrographs are shown in the lower part.

The bottom right part of FIG. 9 is the result of the human colon organoid cultured in the presence of the flow of the culture medium. The hematoxylin and eosin-stained images of the cross sections of the two-dimensional organoids are shown in the upper part, and the micrographs are shown in the lower part.

As a result, it was clarified that, in the two-dimensionally cultured small intestinal (duodenal) organoids, the formation of a villus structure was promoted in the presence of the flow of the culture medium. On the other hand, it was clarified that, in the two-dimensionally cultured colon organoids, no villus structure was formed even in the presence of the flow of the culture medium.

In addition, as a result of performing the same experiment using a two-dimensionally cultured ileal organoid, it was clarified that the same result as that of the two-dimensional duodenal organoid was obtained and the formation of a villus structure was promoted in the presence of the flow of the culture medium.

FIG. 10 is a 3D image showing the result of culturing the two-dimensionally cultured duodenal organoid for 4 days in the presence of the flow of the culture medium, then, fixing the organoid, staining nuclei with Hoechst33342, capturing the nuclei with a two-photon microscope, and performing a coloring process based on the height. As a result, it was confirmed that a villus-like structure was formed in vitro.

### [Experimental Example 5]

### (RNAseq analysis of human duodenal organoid)

Two-dimensionally cultured human duodenal organoids were cultured for 4 days in the presence and absence of the flow of a culture medium, then, RNA was collected, and RNAseq analysis was performed. A sequence library was made using TruSeq RNA Library Prep Kit v2 (Illumina, Inc.). Sequencing was performed using HiSeq X Ten (Illumina, Inc.).

A read was aligned to hg38, which is a reference human genomic sequence, using STAR (version 2.6. 1b) software, and the gene expression level was estimated using RSEM (version 1.3.3) software. Gene expression analysis was performed using RBioconductor package DESeq2.

FIG. 11 is an MA plot showing the results of the RNAseq analysis. In FIG. 11, dotted lines indicate that the values of log2 (fold change) are 1 and -1. In FIG. 11, "Enterocyte" indicates an absorptive epithelial cell marker, "Secretory" indicates an endocrine marker, and "Stem" indicates a stem cell marker.

As a result, it was clarified that, compared with the absence of the flow of the culture medium, the expression of the absorptive epithelial cell markers was elevated in the presence of the flow of the culture medium.

### [Experimental Example 6]

### (Analysis of two-dimensionally cultured ileal organoid)

Two-dimensionally cultured human ileal organoids were cultured for 4 days in the presence and absence of the flow of a culture medium, then, fixed and embedded in paraffin, and subjected to immunostaining. DAB staining was performed using, as primary antibodies, a rabbit anti-SLC10A2 antibody (1: 1000, catalog number "HPA004795", Atlas Antibodies AB) and a mouse anti-sucrase-isomaltase antibody (1:50, catalog number "sc-393470", Santa Cruz Biotechnology, Inc.). SLC10A2 (also called IBAT) and sucrase-isomaltase are small intestine-specific markers.

FIG. 12 is photographs showing the results of the immunostaining. The upper part of FIG. 12 shows the results of detecting the expression of sucrase-isomaltase in the cross sections of the two-dimensionally cultured human ileal organoids. "Flow (-)" indicates that the photograph is the result in the absence of the flow of the culture medium, and "Flow (+)" indicates that the photograph is the result in the presence of the flow of the culture medium. The scale bar is 200 µm.

The upper part of FIG. 12 shows the results of detecting the expression of IBAT (SLC10A2) in the cross sections of the two-dimensionally cultured human ileal organoids. "Flow (-)" indicates that the photograph is the result in the absence of the flow of the culture medium, and "Flow (+)" indicates that the photograph is the result in the presence of the flow of the culture medium. The scale bar is 200 µm.

As a result, it was clarified that, in the organoid having a flat structure obtained in the absence of the flow of the culture medium, neither the expression of the sucrase-isomaltase nor the expression of IBAT (SLC10A2) was recognized.

In contrast, it was clarified that, in the presence of the flow of the culture medium, the formation of a villus structure was recognized and the expression of the sucrase-isomaltase and IBAT (SLC10A2) were recognized in the villus structure part.

Subsequently, two-dimensionally cultured human ileal organoids were cultured for 4 days in the presence and absence of the flow of a culture medium, then, fixed and observed with an electron microscope. Electron micrographs were captured as described below. First, the two-dimensionally cultured human ileal organoids were fixed by being immersed in 2% glutaraldehyde overnight at 4°C. Subsequently, samples were fixed in 2% OsO4 for 2 hours, dehydrated stepwise in a series of ethanol and embedded in Epon812. Subsequently, semi-thin sections were stained with toluidine blue, and additional trimming for a transmission electron microscope was guided. Subsequently, ultrathin sections (80 to 90 nm) were made using an ultramicrotome and mounted on copper grids. Subsequently, the sections were double-stained with uranyl acetate and lead citrate, and electron micrographs were obtained at 100 kV using an H-7600 TEM (Hitachi, Ltd.). All of the electron micrographs were recorded using an XR16S-R CCD camera (Advanced Microscopy Techniques, Corp.).

FIG. 13 is captured electron micrographs. In FIG. 13, "Flow (+)" indicates that the photograph is the result in the presence of the flow of the culture medium, and "Flow (-)" indicates that the photograph is the result in the absence of the flow of the culture medium. The scale bar in the left photograph is 4 µm, and the scale bar in the right photograph is 1 µm.

In addition, FIG. 14 is a graph showing the lengths of microvilli measured based on the electron micrographs of FIG. 13. In FIG. 14, the vertical axis indicates the microvilli length (µm). In addition, "Flow (-)" indicates that the photograph is the result in the absence of the flow of the culture medium, and "Flow (+)" indicates that the photograph is the result in the presence of the flow of the culture medium. In addition, "bottom" indicates the results of the base parts (flat parts) of the villus structures, and "top" indicates the results of the top parts of the villus structures. In addition, "***" indicates that p < 0.001 and there is a significant difference.

As a result, it was clarified that the development of the microvillus was significantly promoted in the top part of the villus structure in the presence of the flow of the culture medium.

### [Industrial Applicability]

According to the present invention, it is possible to provide a technique for producing a two-dimensional small intestinal organoid having a villus structure and an organoid for a small intestinal disease treatment.

## Claims

1. A method for producing a two-dimensional small intestinal organoid having a villus structure, the method comprising:
a step 1 of culturing a cell derived from a small intestinal epithelium in an extracellular matrix to obtain a three-dimensional small intestinal organoid;
a step 2 of dispersing the three-dimensional small intestinal organoid and monolayer culturing on the extracellular matrix to obtain a two-dimensional small intestinal organoid; and
a step 3 of further culturing the two-dimensional small intestinal organoid while letting a culture medium of the two-dimensional small intestinal organoid to flow so that the two-dimensional small intestinal organoid forms a villus structure.

2. A two-dimensional small intestinal organoid having a villus structure.

3. The two-dimensional small intestinal organoid according to Claim 2, wherein the two-dimensional small intestinal organoid is produced from a cell derived from a patient with a small intestinal dysfunction or a small intestinal disease.

4. The two-dimensional small intestinal organoid according to Claim 2, wherein the two-dimensional small intestinal organoid is produced from a cell derived from a normal small intestine.

5. A method for screening an absorption controller of a nutrient or a drug in a small intestine, the method comprising:
a step of measuring an absorption amount of a nutrient or a drug by a two-dimensional small intestinal organoid having a villus structure in a presence of a test sub stance,
wherein a case where the absorption amount is significantly changed compared with that in an absence of the test substance indicates that the test substance is an absorption controller of a nutrient or a drug in a small intestine.

6. A method for screening a formation controller of a villus structure in a small intestine, the method comprising:
a step of producing a two-dimensional small intestinal organoid having a villus structure in a presence of a test substance; and
a step of evaluating a morphology of the villus structure of the produced two-dimensional small intestinal organoid,
wherein a case where the morphology is significantly changed compared with that in an absence of the test substance indicates that the test substance is a formation controller of a villus structure in a small intestine.

7. A method for screening a regulator of digestion, absorption, or metabolism of a target substance in a small intestine, the method comprising:
a step of producing a two-dimensional small intestinal organoid having a villus structure in a presence of a test substance; and
a step of measuring digestion, absorption, or metabolism of a target substance in the produced two-dimensional small intestinal organoid to obtain a measurement value,
wherein a case where the measurement value is significantly changed compared with that in an absence of the test substance indicates that the test substance is a regulator of digestion, absorption, or metabolism of the target substance in a small intestine.

8. A method for screening a substance that regulates growth of an intestinal bacterium and/or a virus in a small intestine, the method comprising:
a step of measuring growth of an intestinal bacterium and/or a virus in a two-dimensional small intestinal organoid having a villus structure in a presence of a test sub stance,
wherein a case where the growth of the intestinal bacterium and/or virus is significantly changed compared with that in an absence of the test substance indicates that the test substance is a substance that regulates growth of an intestinal bacterium and/or a virus in a small intestine.

9. A transplant material containing a small intestinal organoid, the transplant material is for being transplanted into a large intestine of a living body to cause an intestinal fluid to flow so that a tissue having a small intestinal function is formed in the large intestine.

10. The transplant material according to Claim 9, wherein the transparent material is for being transplanted after an epithelium of the large intestine is peeled off and removed.

11. A transplant material containing a small intestinal epithelial organoid, the transplant material is for being transplanted after an epithelium of a large intestine of a living body is peeled off and to cause an intestinal fluid to flow so that a tissue having a small intestinal function is formed in the large intestine.

12. A treatment method of a small intestinal dysfunction, the treatment method comprising:
transplanting an effective amount of a small intestinal organoid into a large intestine of a patient in need of treatment so as to cause an intestinal fluid flow to form a tissue having a small intestinal function in the large intestine.
